# EUROPÄISCHE PATENTSCHRIFT

(11) **EP 1 369 094 B1**
(45) Veröffentlichungstag und Bekanntmachung des Hinweises auf die Patenterteilung: **26.11.2014**
(21) Anmeldenummer: 02012124.0
(22) Anmeldetag: 31.05.2002
(51) Int. Cl.: A61F 2/00, A61L 27/16

(54) **Implantat und Verfahren zur Herstellung eines steril verpackten Implantats**
Implant and method of manufacturing a sterile packaged implant
Implant et procédé pour fabriquer un implant emballé stérilement

(43) Veröffentlichungstag der Anmeldung: 10.12.2003
(73) Patentinhaber: Zimmer GmbH, 8404 Winterthur (CH)
(72) Erfinder: Baege, Roland, 8542 Wiesendangen (CH); Breimesser, Hermann, 8353 Elgg (CH); Willi, Roland, 8413 Neftenbach (CH)
(74) Vertreter: Manitz, Finsterwald & Partner GbR

(56) Entgegenhaltungen:
- EP-A- 0 923 945
- WO-A-01/80778
- GB-A- 1 489 642
- US-A- 3 932 898
- US-A- 6 165 220
- STREICHER R M: "INVESTIGATION ON STERILIZATION AND MODIFICATION OF HIGH MOLECULAR WEIGHT POLYETHYLENES BY IONIZING IRRADIATION" REPRINT FROM BETA-GAMMA, XX, XX, Nr. 1, Januar 1989 (1989-01), Seiten 1-10, XP002924952

## Beschreibung

Die Erfindung betrifft ein Implantat, sowie ein Verfahren zum Bereitstellen einer Anordnung aus einem Implantat und einer Verpackung.

Ultrahochmolekulares Polyethylen (UHMWPE) ist ein in der orthopädischen Endoprothetik verbreiteter Werkstoff, der sich als Gleitpartner gegenüber metallischen und keramischen Werkstoffen bewährt hat. Problematisch ist bei diesem Materialpaarungen der im Gebrauch unvermeidliche Abrieb und die dadurch ausgelöste partikelinduzierte Osteolyse, die schlimmstenfalls zu einer Lockerung des Implantats führen kann.

Eine geeignete Maßnahme zur Optimierung des UHMWPE mit dem Ziel der Reduktion des entstehenden Abriebs ist die Hochvernetzung. Diese wird durch Elektronen- oder Gammabestrahlung und eine Wärmebehandlung des Materials erreicht, wodurch das ultrahochmolekulare Polyethylen verschleißfest und langzeitstabil gemacht wird. Die Hochvernetzung erfolgt durch eine Vernetzung der UHMWPE-Molekülketten und durch eine Absättigung der freien Radikale.

Die in der orthopädischen Endoprothetik übliche Sterilisationsmethode der Gammabestrahlung kann für hochvernetztes UHMWPE nicht angewendet werden, weil dadurch wieder unerwünschte freie Radikale entstehen würden. Diese begünstigen eine Oxidationswirkung und eine damit verbundene Versprödung des Materials. Daher wird hochvernetztes UHMWPE nicht mit ionisierender Strahlung, sondern mit Gas, beispielsweise mit Ethylenoxid oder Gas-Plasma, sterilisiert.

Bei Implantatkomponenten, die ausschließlich aus hochvernetztem Polyethylen bestehen, werden mittels Hochvernetzung und Wärmebehandlung des UHMWPE-Rohlings, anschließender mechanischer Bearbeitung, Verpackung und Gassterilisation zufrieden stellende Ergebnisse erzielt.

Jedoch können Implantate aus mehreren, während des Herstellprozesses zusammengesetzten Elementen bestehen. Bei derartigen, zusammengesetzten Implantaten stellt sich die Problematik der wirksamen Sterilisation. Eine Sterilisation mit Gammastrahlen würde das gesamte Implantat erfassen und somit Elemente aus UHMWPE oxidieren. Eine Gassterilisation hingegen wirkt nur auf all diejenigen Oberflächen des Implantates, die für das Gas während der Sterilisation zugänglich sind. Um dabei auch durch Zusatzelemente abgedeckte Oberflächen für das Sterilisiergas zugänglich zu machen, ist es bekannt, die Verbindung von Implantatelementen mit Spiel vorzunehmen. Aufgrund klinischer Erfahrung ist jedoch ein solches Spiel problematisch, da auf diese Weise ermöglichte Mikrobewegungen von Implantatelementen im Verdacht stehen, unerwünschten Abrieb zu erzeugen und einem dauerhaften Anwachsen des Knochens entgegen zu wirken.

US 3,932,898 offenbart eine prothetische Herzklappe mit einem Grundkörper und einem damit verrasteten Käfig.

Aufgabe der Erfindung ist es, ein mehrteiliges Implantat anzugeben, das eine wirksame Sterilisation und Aufrechterhaltung des sterilen Zustands bis zum praktischen Einsatz des Implantats ermöglicht.

Diese Aufgabe wird durch die Merkmale des Anspruchs 1 gelöst. Bei einem Implantat mit wenigstens einem ersten Element und einem zweiten Element, wobei das erste Element in einer vorgegebenen Relativlage zum zweiten Element vorgesehen ist, ist vorgesehen, dass das erste Element bezüglich des zweiten Elements eine spielbehaftete, eine vollflächige Gassterilisation beider Elemente gewährleistende Vorpositionierungsstellung aufweist und aus dieser Vorpositionierungsstellung in eine einem Festverbund beider Elemente entsprechende Endpositionierungsstellung verlagerbar ist.

Wird das mehrteilige Implantat in einer vorgegebenen Vorpositionierungsstellung für das erste Element verpackt und wird anschließend eine Gassterilisation durchgeführt, während sich das erste Element in der definierten Vorpositionierungsstellung befindet, kann eine außerordentlich wirksame, vollflächige Sterilisation des mehrteiligen Implantats erreicht werden. Das sterilisierte Implantat wird in dieser vormontierten Position in der Verpackung transportiert und gelagert, bis es bei Bedarf bis an das Operationsfeld gebracht wird, wo es im vormontierten Zustand nach dem Aufreißen der Verpackung zur Verfügung steht.

Mit dem erfindungsgemäßen Implantat wird sichergestellt, dass die zeitliche Differenz zwischen der Sterilisation und der Operation keine nachteiligen Auswirkungen haben kann. Das zum Zeitpunkt der Gassterilisation und während der Lagerung bzw. dem Transport vorhandene Spiel zwischen den mechanisch mit einander verbundenen Einzelelementen kann kurz vor dem Implantieren einfach durch Verlagerung des ersten Elements von der Vorpositionierungsstellung in die Endpositionierungsstellung aufgehoben und das erste Element zuverlässig bezüglich des zweiten Elements für die Implantierung fixiert werden. So erübrigt sich auch ein umständliches Auspacken und Zusammensetzen von Einzelelementen des Implantats, wobei das Implantat kontaminiert werden könnte.

Ferner kann in einer Ausführungsform des erfindungsgemäßen Implantats, bei der das erste Element zumindest teilweise in einer Ausnehmung des zweiten Elements vorgesehen ist, das erste Element in der Vorpositionierungsstellung in der Ausnehmung lose gehalten sein, wobei die Berührungsstellen zwischen dem ersten Element und dem zweiten Element in der Ausnehmung kleinflächig sind, und in einer Endpositionierungsstellung in der Ausnehmung mit dem zweiten Element kraft- und formschlüssig verbunden sein. Das erste Element ist dabei in der Ausnehmung von der Vorpositionierungsstellung in die Endpositionierungsstellung verlagerbar.

Dadurch, dass in der Vorpositionierungsstellung das erste Element in der Ausnehmung lose gehalten ist und die Berührungsstellen zwischen dem ersten Element und dem zweiten Element in der Ausnehmung kleinflächig sind, sind praktisch alle Oberflächen des ersten und des zweiten Elements vom Sterilisiergas erreichbar. Gleichzeitig ist das Spiel in der Vorpositionierungsstellung ausreichend, um die Oberflächen des Implantats dem Sterilisationsgas während dem Sterilisationsprozess zugänglich zu machen, jedoch klein genug, um einen unerwünschten Abrieb bei Erschütterungen des Implantats zu vermeiden. Zudem ermöglicht die kraft- und formschlüssige Verbindung der Elemente in der Endpositionierungsstellung einen sicheren und funktionsgerechten Gebrauch des eingesetzten Implantats.

Das Material mindestens eines der ersten und zweiten Elemente kann hochvernetztes ultrahochmolekulares Polyethylen und/oder einen anderen Kunststoff umfassen. Diese Materialien sind verwendbar, weil das erfindungsgemäße Implantat eine außerordentlich wirksame Gassterilisation derartig vormontierter Kunststoff-Teile ermöglicht.

Das Material eines der ersten und zweiten Elemente kann ferner ein Metall, insbesondere Titan, umfassen. Demnach können Implantate, die Elemente unterschiedlicher Materialkombinationen aufweisen, erfindungsgemäß ausgebildet und damit auch zuverlässig mit Gas sterilisiert werden.

In einer Ausführungsform kann das erste Element in der Vorpositionierungsstellung im wesentlichen zentriert lose gehalten sein. Diese Zentrierung ermöglicht ein zu allen Seiten gleich geringes Spiel zwischen den Elementen, so dass alle Oberflächen des Implantats während der Gassterilisation vom Sterilisationsmedium erreichbar sind.

Das erste Element kann in der Vorpositionierungsstellung mit einem Spiel von mindestens 0,1 mm, beispielsweise 0,2 bis 0,4 mm, gehalten sein. Dieses Spiel ermöglicht die Zugänglichkeit praktisch aller Oberflächen des Implantats und damit eine sichere Gassterilisation, eine sterile Lagerung und einen sterilen Transport im verpackten Zustand.

Die Ausnehmung kann einen im wesentlichen kreisförmigen Querschnitt aufweisen, das erste Element kann einen im wesentlichen kreisförmigen und/oder im wesentlichen sternförmigen Querschnitt aufweisen, und das erste Element kann in der Vorpositionierungsstellung in der Ausnehmung radial und axial lose gehalten sein. So wird erreicht, dass bei einer Ausnehmung und einem ersten Element mit kreisförmigen Querschnitten zwischen dem ersten und dem zweiten Element sowohl radial als auch axial ein Spiel vorhanden ist, das eine wirksame Gassterilisation aller Oberflächen des Implantats ermöglicht.

Insbesondere können die Berührungsstellen zwischen dem ersten Element und dem zweiten Element in der Ausnehmung im wesentlichen punktförmig sein. Dadurch wird die Größe der Kontaktflächen zwischen dem ersten und dem zweiten Element minimiert, so dass unter Berücksichtigung der geringen relativen Beweglichkeit zwischen den Elementen alle Oberflächen des Implantats von dem Sterilisationsmedium während der Gassterilisation umströmt werden können.

Ferner wird die Anzahl der Berührungsstellen in der Ausnehmung zwischen dem ersten Element und dem zweiten Element in der Vorpositionierungsstellung gering gehalten. Auch dies führt dazu, dass die Kontaktfläche zwischen dem ersten und dem zweiten Element möglichst klein gehalten wird, so dass das Implantat optimal vom Sterilisationsmedium umspült werden kann.

In einer Ausführungsform wird das erste Element in der Vorpositionierungsstellung mittels einer Schnappverbindung lose gehalten und in der Endpositionierungsstellung mit dem zweiten Element im Klemmsitz und/oder mittels einer Schnappverbindung kraft- und formschlüssig verbunden. Dadurch kann das erste Element durch eine einfache Verlagerung, wie z.B. eine Verdrehung und/oder Verschiebung von der Vorpositionierungsstellung in die Endpositionierungsstellung überführt werden, in der das erste Element in dem zweiten Element fixiert ist.

Ferner kann das erste Element mindestens einen Vorsprung aufweisen, der in der Endpositionierungsstellung in das zweite Element eingreift. Dies gewährleistet eine sichere kraft- und formschlüssige Verbindung des ersten Elements mit dem zweiten Element des Implantats.

Außerdem kann das erste Element eine Kugel oder ein Stift, insbesondere eine Metallkugel oder ein Metallstift, sein. So können beispielsweise Röntgenkontrastkugeln oder -stifte in einem Implantat wirksam mit Gas sterilisiert und vor der Operation zuverlässig im Implantat fixiert werden.

Das erste Element kann eine Kugel und einen Stopfen mit einer Aufnahme für die Kugel umfassen. In der Vorpositionierungsstellung kann die Kugel in dem Stopfen oder zwischen dem Stopfen und der Ausnehmung lose gehalten sein, und in der Endpositionierungsstellung kann der Stopfen in der Ausnehmung und die Kugel in dem Stopfen kraft- und formschlüssig gehalten sein. Mit dieser Ausführungsform können winzige Kugeln, beispielsweise mit einem Durchmesser von 2 mm zusammen mit dem Implantat zuverlässig sterilisiert und intraoperativ zuverlässig in dem Implantat befestigt werden. Gleichzeitig wird die Handhabung der Kugeln und ihr Sterilhalten während der Handhabung vereinfacht.

In einer weiteren Ausführungsform kann das zweite Element eine Innenschale einer Gelenkprothese und das erste Element eine Abdeckscheibe der Innenschale sein. Die Ausnehmung kann in der Außenwand der Innenschale mit einem kreisförmigen Querschnitt, einer Seitenwand und einem zum Zentrum der Ausnehmung tiefer werdenden Boden vorgesehen sein, wobei die Seitenwand mindestens einen zum Zentrum der Ausnehmung gerichteten Haltevorsprung aufweist. Die Abdeckscheibe kann von der Seitenwand und dem Vorsprung lose gehalten werden und kann mindestens einen Nocken an ihrem Außenumfang aufweisen, die in der Endpositionierungsstellung in die Seitenwand der Ausnehmung klemmend eingreift, wenn die Abdeckscheibe durch eine Drehbewegung von der Vorpositionierungsstellung in die Endpositionierungsstellung verlagert wird.

Dieser Aufbau ermöglicht beispielsweise bei einer kegelstumpfförmigen Hüftgelenkprothesenschale mit Abdeckscheibe eine hochwirksame Sterilisation mit Gas, da die Abdeckscheibe in der Vorpositionierungsstellung mit einem ausreichenden Spiel gelagert ist. Durch den zum Zentrum der Ausnehmung tiefer werdenden Boden, der ebenen Form der Abdeckscheibe und den Vorsprung wird der Kontakt zwischen dem ersten Element und dem zweiten Element kleinflächig gehalten. Das Gas erreicht den Boden durch Nuten, welche die Seitenwand unterbrechen und bis an den Boden reichen. So wird eine allseitige Umströmung der Abdeckscheibe in der Ausnehmung während der Gassterilisation erreicht. Der wenigstens eine Nocken der Abdeckscheibe gewährleistet zudem eine außerordentlich feste Verbindung zwischen dem ersten und dem zweiten Element in der Endpositionierungsstellung.

In der Seitenwand der Ausnehmung kann mindestens eine Aufnahme für den Nocken in der Vorpositionierungsstellung mit Spiel vorgesehen sein. Dadurch wird ermöglicht, dass auch der Nockenbereich während der Gassterilisation in der Vorpositionierungsstellung vom Sterilisationsmedium ausreichend umströmt wird.

Außerdem kann die Seitenwand der Ausnehmung eine Führung, insbesondere eine Einschubführung, zum Einbringen des ersten Elements in die Vorpositionierungsstellung aufweisen. Dies erleichtert die Montage des ersten Elements in dem zweiten Element vor der Durchführung der Gassterilisation.

Die Lösung der Aufgabe erfolgt außerdem durch eine Anordnung aus einem Implantat und einer Verpackung gemäß Anspruch 22 sowie ein Verfahren zum Bereitstellen eines Implantats und einer Verpackung gemäß Anspruch 23 insbesondere dadurch, dass zur Herstellung eines sterilisiert verpackten Implantats mit wenigstens einem ersten Element und einem zweiten Element die das Implantat bildende Elemente in einer gegenseitig gekoppelten, relativ zueinander Spiel aufweisenden Vorpositionierungsstellung mit Gas sterilisierbar sind und in dieser Vorpositionierungsstellung bis in das eigentliche Operationsfeld einbringbar sind.

Die Gassterilisation kann mit Ethylenoxid durchgeführt werden.

Die in einer Vorpositionierungsstellung steril verpackten Einzelelemente können nach Öffnen der Verpackung durch Dreh- und/oder Schiebebewegungen zur Fertigmontage des Implantats miteinander form- und kraftschlüssig verbunden werden.

Weitere vorteilhafte Ausführungsformen der Erfindung sind in der Beschreibung, der Zeichnung und den Unteransprüchen beschrieben.

Nachfolgend wird die Erfindung rein beispielhaft unter Bezugnahme auf die beigefügten Zeichnungen beschrieben. Es zeigen:
- Fig. 1a, 1b: eine Querschnittsansicht einer ersten Ausführungsform eines erfindungsgemäßen Implantats und eine zugehörige perspektivische Draufsicht,
- Fig. 2a - 2d: Querschnittsansichten einer zweiten Ausführungsform,
- Fig. 3a - 3c: Querschnittsansichten einer dritten Ausführungsform,
- Fig. 4a - 4c: Querschnittsansichten einer vierten Ausführungsform,
- Fig. 5a - 5c: Querschnittsansichten einer fünften Ausführungsform,
- Fig. 6a - 6c: Querschnittansichten einer sechsten Ausführungsform,
- Fig. 7a - 7c: Querschnittsansichten einer siebten Ausführungsform,
- Fig. 8a - 8c: Querschnittsansichten einer achten Ausführungsform,
- Fig. 9a - 9c: Querschnittsansichten einer neuen Ausführungsform,
- Fig. 10a - 10d: Querschnittsansichten einer zehnten Ausführungsform.

### Erste Ausführungsform

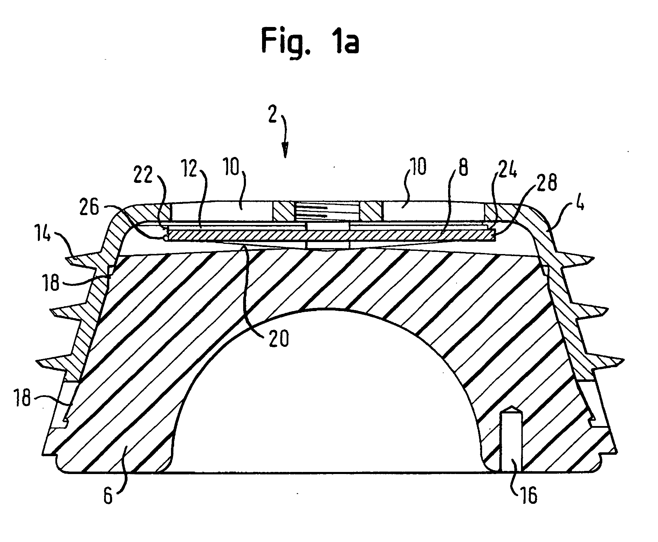

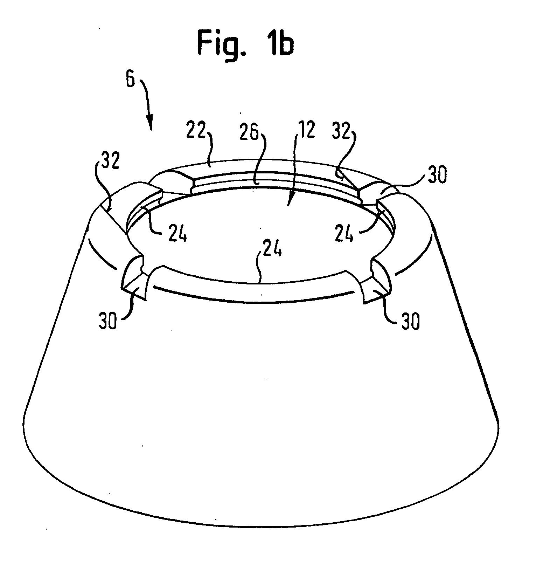

Eine in Fig. 1a und 1b in der Endpositionierungsstellung gezeigte erste Ausführungsform des erfindungsgemäßen Implantats besteht aus einer kegelstumpfförmigen Hüftgelenksprothesenschale 2 mit einer Außenschale 4 aus Titan, einer Innenschale 6 aus hochvernetztem ultrahochmolekularen Polyethylen (UHMWPE) und einem Abdeckblech 8 aus Titan. Die Hüftgelenksprothesenschale 2 ist als Hüftpfanne ausgebildet, die ohne Verwendung von Knochenzement implantiert wird. Öffnungen 10 in der Außenschale erlauben es, den Sitz der Schale im Knochen zu kontrollieren und eventuelle Hohlräume mit Knochenspänen zu füllen. Um einen direkten Kontakt des Knochens mit dem Polyethylen der Innenschale 6 durch die Öffnungen 10 der Außenschale zu verhindern, ist das Abdeckblech 8 aus dem biokompatiblen Material Titan vorgesehen. Dieses befindet sich in einer Ausnehmung 12 der Innenschale und ist in dieser Ausnehmung 12 zentriert.

Wie aus Fig. 1a ersichtlich ist, weist die Außenschale 4 ein Schneidgewinde 14 zur Verankerung der Hüftpfanne im Knochen auf. An der Unterseite der Innenschale 6 sind Bohrungen 16 vorgesehen, die zum Einsetzen eines Werkzeugs dienen. Die Innenschale 6 wird in der Außenschale 4 mittels einer Schnappverbindung 18, bestehend aus Vertiefungen auf der Innenseite der Außenschale 4 und Vorsprüngen auf der Außenseite der Innenschale 6, befestigt.

Fig. 1a zeigt, dass die Ausnehmung 12 einen Schrägboden 20 besitzt, der zum Zentrum der Innenschale 6 abgesenkt ist. Die Ausnehmung 12 wird von einer Seitenwand 22 begrenzt, an deren oberen Ende etwa in einer Umfangshälfte der Ausnehmung ein nach innen gerichteter Vorsprung 24 vorgesehen ist. Dieser Vorsprung 24 übergreift das Abdeckblech 8, wenn dieses von der dem Vorsprung 24 gegenüberliegenden Seite her in die Ausnehmung 12 eingeführt wird.

Fig. 1b zeigt eine perspektivische Seitenansicht der Innenschale, in der das Abdeckblech nicht eingesetzt ist. Wie daraus ersichtlich ist, sind in der Seitenwand 22 vier zueinander um 90° versetzt angeordnete, radial verlaufende Nuten 30 vorgesehen. Zusätzlich sind angrenzend an die Enden des Vorsprungs 24 zwei Einschubführungen 32 für das Abdeckblech 8 vorgesehen. Die Einschubführungen 32 sind derart ausgebildet, dass sie das Abdeckblech 8 beim Einsetzen in die Ausnehmung 12 unter den Vorsprung 24 lenken.

In der Ausnehmung 12 ist ferner im Übergang zwischen der Seitenwand 22 und dem Boden 20 eine umlaufende Rille 26 vorgesehen.

Das eben ausgebildete Abdeckblech 8 weist an seinem Umfang einen Eingriffsnocken 28 auf. In Fig. 1a ist dieser eine Nocken 28 erkennbar.

Beim Einsetzen des Abdeckblechs 8 in die Ausnehmung 12 wird das Abdeckblech zwischen den Einschubführungen 32 auf die Seitenwand 22 aufgesetzt und in die Ausnehmung 12 eingeschoben, wobei der Nocken 28 in eine Nute 30 zu liegen kommt. Das Abdeckblech 8 wird dabei unter den Vorsprung 24 in der Ausnehmung 12 geschoben und nimmt dann die Vorpositionierungsstellung ein, in der es durch den Vorsprung 24 lose gehalten wird. Die Abmessungen der Ausnehmung 12, das Vorhandensein der Rille 26 und der Schrägboden 20 bewirken, dass das Abdeckblech 8 in der Ausnehmung einerseits mit einem Spiel von beispielsweise etwa 0,2 mm gehaltert und andererseits ausschließlich kleinflächig abgestützt ist. Der Nocken 28 der Abdeckscheibe ist dabei in einer der Nuten 30 der Seitenwand 22 lose positioniert, d.h. es liegt die Vorpositionierungsstellung vor.

Zum festen, d.h. kraft- und formschlüssigen Verbinden des Abdeckblechs 8 mit der Innenschale 6 wird das Abdeckblech 8 unter Zuhilfenahme eines Werkzeugs derart verdreht, dass sich der Nocken 28 in die Seitenwand 22 eingräbt und mit dieser in der Endpositionierungsstellung des Abdeckblechs 8 unverrückbar verbunden ist. Dieser Zustand ist in Fig. 1a dargestellt.

Zur Gewährleistung des sicheren Erreichens der Endpositionierungsstellung dienen in Fig. 1a, 1b nicht dargestellte, außen auf dem Abdeckblech 8 angebrachte Markierungen, die in der Vorpositionierungsstellung des Abdeckblechs drehversetzt zu den Nuten 30 angeordnet sind. Um das Abdeckblech 8 in die Endpositionierungsstellung zu bringen, wird das Werkzeug in das Abdeckblech 8 eingesetzt und das Blech gedreht, bis die Markierungen auf dem Abdeckblech 8 und die Nuten 30 einander direkt gegenüberliegen.

Die Vorpositionierungsstellung des Abdeckblechs 8 wird vor der Sterilisation der Innenschale mit Ethylenoxid beim Hersteller des Implantats eingestellt. Anschließend wird die Gassterilisation durchgeführt. Da in der Vorpositionierungsstellung die Kontaktflächen zwischen dem Abdeckblech 8 und der Innenschale 6 in der Ausnehmung 12 aufgrund des geringen Spiels und des Schrägbodens 20 der Ausnehmung auf einem Minimum gehalten sind, kann das Ethylenoxid unter Berücksichtigung der relativen Beweglichkeit der Einzelteile bei der Sterilisation praktisch alle Oberflächen des Abdeckblechs 8 und der Innenschale 6 umströmen. So wird eine außerordentlich wirksame und sichere Gassterilisation ermöglicht.

Zur bekannten Gassterilisation sind die Teile bereits sauber verpackt in mehreren ineinander liegenden Verpackungen eingebracht, welche durch gasdurchlässige Membranen verschlossen sind. Diese Membranen lassen wohl Gasmoleküle, jedoch keinerlei Mikroorganismen passieren. Die so verpackten Teile werden in einem abgeschlossenen Behälter mit verschiedenen Gasen bei unterschiedlichen Drücken gespült, wobei ein eigentliches toxisches Sterilisiergas, z.B. Ethylenoxid bei hohem Druck an den Teilen anliegt, um Sterilität durch Abtötung von Mikroorganismen zu erreichen. Dieses toxische Gas muss anschließend mit nachgeschalteten Spülvorgängen und bei Unterdruck durch die Membranen hindurch abgezogen werden. Am Ende des Sterilisationsvorgangs liegen die sterilen Teile von Luft umgeben in einer sterilen Verpackung.

Während der Sterilisation der in der Vorpositionierungsstellung verpackten Innenschale 6 ermöglicht das Spiel zwischen dem Abdeckblech 8 und der Innenschale 6, dass das Implantat dem Sterilisationsgas vollflächig ausgesetzt ist. Zusätzlich wird durch das klein gehaltene Spiel vermieden, dass das Implantat durch Bewegungen beim Transport beeinträchtigt wird.

Die Verpackung wird kurz vor dem Implantieren bzw. dem Einsetzen der Innenschale 6 in die bereits implantierte Außenschale 4 der Hüftgelenksprothese 2 geöffnet, und das Abdeckblech 8 wird mit Hilfe eines sterilen Werkzeugs in die vorgegebene Endpositionierungsstellung gedreht, in der das Abdeckblech 8 - wie beschrieben - fest mit der Innenschale 6 verbunden ist.

Ein umständliches Einsetzen des Abdeckblechs 8 in die Innenschale 6 im Falle einer separaten Verpackung des Abdeckblechs 8 mit der Gefahr der Kontaminierung des Implantats wird somit vermieden.

Nach Einstellen der Endpositionierungsstellung des Abdeckblechs 8 in der Innenschale 6 wird diese in die Außenschale unter Zuhilfenahme eines Werkzeugs eingesetzt, wobei die Schnappverbindung 18 wirksam wird.

Auf diese Weise wird sichergestellt, dass die in einer Vorpositionierungsstellung zusammengebrachten Teile trotz Sterilisation, Transport und Auspacken im Operationsbereich nicht mehr voneinander getrennt sind. Eine Zuordnung von Teilen ist nicht mehr notwendig.

### Zweite Ausführungsform

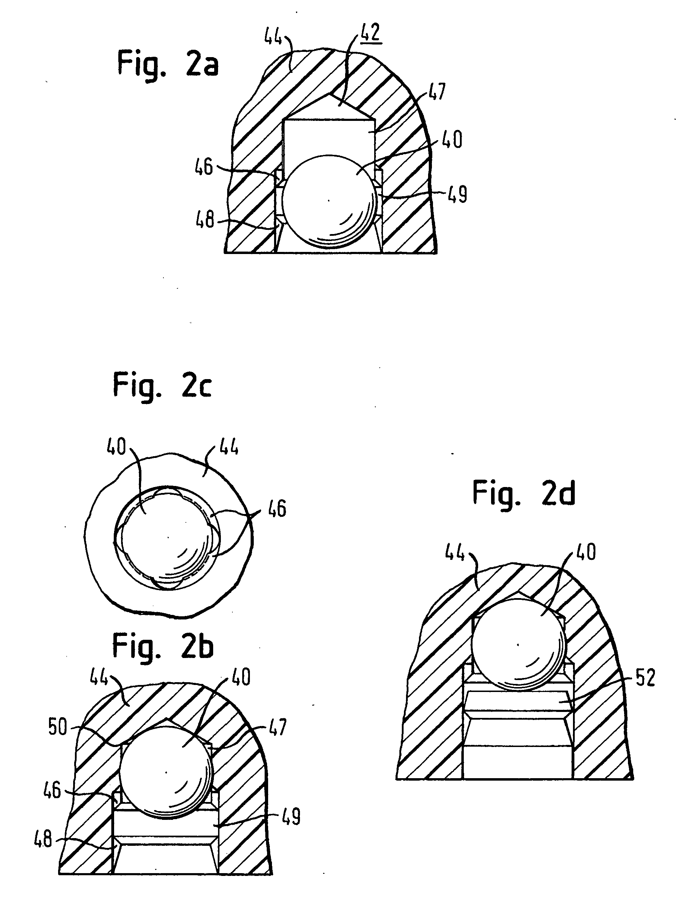

Die in Fig. 2a bis 2d dargestellte zweite Ausführungsform des erfindungsgemäßen Implantats umfasst eine Röntgenkontrast-Kugel 40 aus Metall und ein Element 44 aus hochvernetztem ultrahochmolekularem Polyethylen mit einer Bohrung 42.

Die Bohrung 42 besitzt an ihrem geschlossenen Ende einen Bereich 47 mit einem Durchmesser, der ein kleines Untermaß gegenüber dem Durchmesser der Kugel 40 aufweist. An ihrem offenen Ende weist die Bohrung 42 einen Bereich 49 mit einem Durchmesser auf, der größer als der Durchmesser der Kugel 40 ist.

Im Bereich 49 sind Vorsprünge 46 und 48 vorgesehen, die jeweils ringförmig über den Umfang der Bohrung verteilt sind.

Die in Fig. 2a gezeigte Vorpositionierungsstellung wird beim Hersteller vor der Gassterilisation eingestellt. Dabei ist die Röntgenkontrast-Kugel 40 zwischen den Vorsprüngen 46 und 48 radial und axial lose gelagert. Zudem sind zwischen der Röntgenkontrast-Kugel 40 und den Vorsprüngen 46, 48 nur kleinflächige Berührungsstellen vorhanden. Außerdem liegt zwischen der Kugel 40 und dem Element 44 ein allseitiges Spiel von beispielsweise etwa 0,2 mm vor.

In der Vorpositionierungsstellung wird eine Sterilisation mit Ethylenoxid-Gas durchgeführt, bei der praktisch alle Oberflächen in der Bohrung 42, einschließlich der Röntgenkontrast-Kugel 40 wirksam sterilisiert werden.

Kurz vor dem Einsetzen des Implantats wird es mit der lose gehaltenen Röntgenkontrast-Kugel 40 der Verpackung entnommen, und die Kugel wird zum Ende 50 der Bohrung in die in Fig. 2b und 2c gezeigte Endpositionierungsstellung gedrückt. Dabei ist die Kugel 40 am geschlossenen Ende 50 der Bohrung im Klemmsitz fixiert.

Zum Einpressen der Röntgenkontrast-Kugel 40 kann ein separat gelieferter, steriler Stopfen 52 verwendet werden, der in der Endpositionierungsstellung über Vorsprünge 48 geklemmt und abdichtend in der Bohrung 42 fixiert ist. Wie in Fig. 2d gezeigt, hat der Stopfen 52 eine solche Länge, dass ein bündiges Abschließen des Stopfens 52 mit dem offenen Ende der Bohrung 42 anzeigt, dass die Kugel 40 in ihrer vorgesehenen Endlage fixiert ist.

### Dritte Ausführungsform

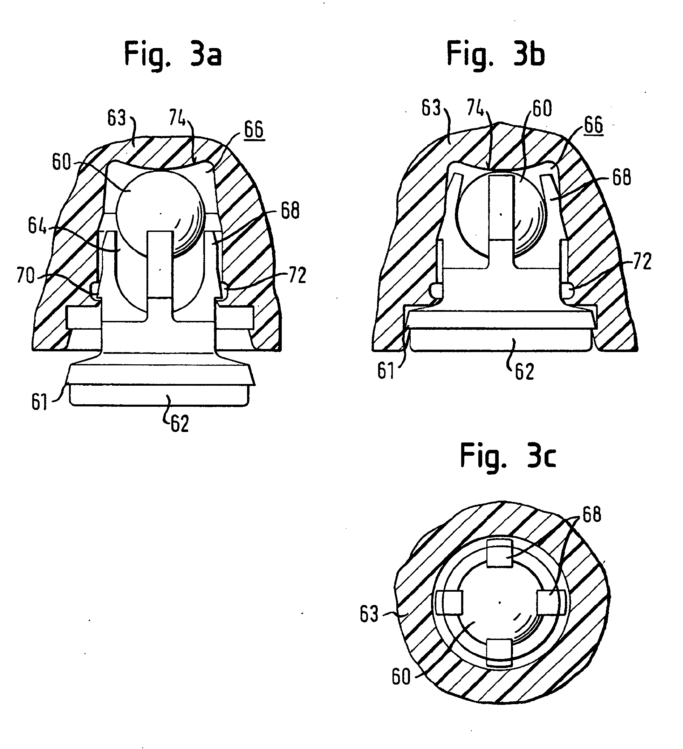

Die in Fig. 3a - 3c gezeigte dritte Ausführungsform umfasst eine Röntgenkontrast-Kugel 60, die zusammen mit einem als Stopfen ausgebildeten Körbchen 62 in einer Bohrung 66 eines Elements eingesetzt wird. Die Röntgenkontrast-Kugel 60 besteht aus Metall, z.B. aus Titan oder Tantal, während mindestens das Material des Elements 63 hochvernetztes ultrahochmolekulares Polyethylen ist.

Das Körbchen 62 besitzt eine Aufnahme 64 mit vier zueinander um 90° versetzt im Kreis angeordneten Armen 68. Die innere Kontur der Arme 68 ist an die Kugeloberfläche der Röntgenkontrast-Kugel angepasst. Auf der Außenseite der Arme 68 ist jeweils ein Vorsprung 70 vorgesehen.

Die Bohrung 66 weist in einem Ring um ihren kreisförmigen Umfang herum angeordnete Vertiefungen 72 zur Aufnahme der Vorsprünge 70 in der Vorpositionierungsstellung auf. Zudem besitzt die Bohrung ein nach innen vorgewölbtes, geschlossenes Ende 74.

In der in Fig. 3a gezeigten Vorpositionierungsstellung ist die Röntgenkontrast-Kugel 60 zwischen dem Körbchen 62 und dem geschlossenen Ende 74 der Bohrung 66 lose angeordnet. Dabei ist das Körbchen 62 mittels einer Schnappverbindung zwischen den Vorsprüngen 70 und den Vertiefungen 72 mit einem allseitigen Spiel von beispielsweise 0,2 mm gehalten. Zwischen der Kugel 60, dem Körbchen 62 und der Bohrung 66 sind nur kleinflächige Kontaktstellen vorhanden.

Die Vorpositionierungsstellung wird beim Hersteller eingestellt. Danach erfolgt eine Gassterilisation mit Ethylenoxid. Diese erfasst das gesamte Implantat, da alle Oberflächen innerhalb der Bohrung durch das offene Ende der Bohrung und durch die Zwischenräume zwischen den Armen 68 vom Gas umströmt werden. Das Körbchen 62 ist in der Vorpositionierungsstellung kraft- und formschlüssig so fest installiert, dass ein Lösen des Körbchens 62 und der Röntgenkontrast-Kugel 60 vom Element 63 während des Transports ausgeschlossen ist.

Die in Fig. 3b und 3c gezeigte Endpositionierungsstellung wird durch Einschieben des Körbchens 62 ausgehend von der Vorpositionierungsstellung zum geschlossenen Ende 74 der Bohrung hin erreicht.

Das Körbchen 62 wird dabei in einem sich verjüngenden Bereich der Bohrung 66 eingeklemmt, während die Kugel 60 zwischen den Armen 68 des Körbchens und dem vorgewölbten Ende 74 der Bohrung kraft- und formschlüssig fixiert ist. Das der Aufnahme 64 entgegengesetzte Ende des Körbchens 62 schließt in der Endpositionierungsstellung mit einer Schnappverbindung 61 bündig mit dem offenen Ende der Bohrung 66 ab.

### Vierte Ausführungsform

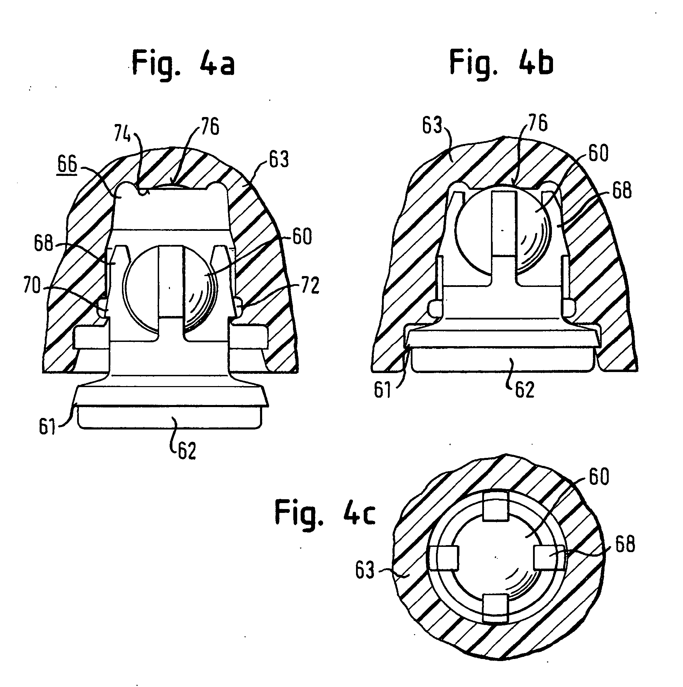

Die vierte Ausführungsform, die in Fig. 4a bis 4c gezeigt ist, entspricht der dritten Ausführungsform, wobei jedoch die Röntgenkontrast-Kugel 60, wie in Fig. 4a gezeigt bereits in der Vorpositionierungsstellung in der Aufnahme 64 des Körbchens gehalten ist. Aufgrund der inneren Kontur der Arme 68 ist dabei zwischen der Kugel 60 und den Armen 68 ein allseitiges Spiel von beispielsweise 0,2 mm vorhanden.

In der Vorpositionierungsstellung wird gewährleistet, dass alle Oberflächen innerhalb der Bohrung unter Berücksichtigung der relativen Beweglichkeit ausreichend vom Sterilisationsgas umströmt werden können. Dabei sind die Kontaktflächen zwischen der Kugel 60 und den Armen 68 kleinflächig. Gleiches gilt für die Kontaktstellen des Körbchens 62 mit dem Implantat 63 in der Schnappverbindung zwischen den Vorsprüngen 70 und den Vertiefungen 72.

Die in Fig. 4b gezeigte Endpositionierungsstellung wird durch eine Verschiebung des Körbchens in Richtung des geschlossenen Endes 74 der Bohrung ausgehend von der Vorpositionierungsstellung eingestellt und ist durch eine Schnappverbindung 61 gesichert. In der Endpositionierungsstellung ist die Kugel 60 und das Körbchen 62 am Ende 74 der Bohrung kraft- und formschlüssig fixiert.

### Fünfte Ausführungsform

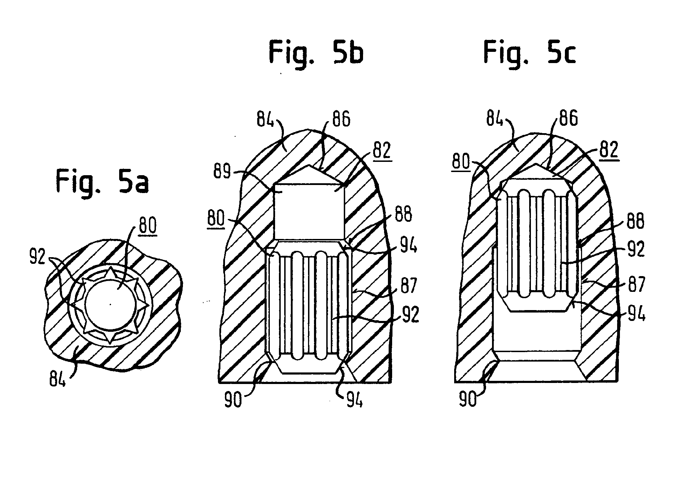

Die fünfte, in Fig. 5a bis 5c gezeigte Ausführungsform umfasst ein Implantat 84 aus hochvernetztem UHMWPE mit einer Bohrung 82 und einen Röntgenkontrast-Stift 80 aus Metall.

Die Bohrung 82 besitzt an ihrem offenen Ende einen Bereich 87 mit einem großen Durchmesser. Am geschlossenen Ende der Bohrung 82 ist ein Bereich 89 mit einem kleineren Durchmesser gebildet. Die Bohrung 82 besitzt ferner zwischen dem Bereich 87 und dem Bereich 89 einen sich verjüngenden Bereich 88 und im Bereich 89 einen zum offenen Ende der Bohrung hin angeordneten ringförmigen Haltevorsprung 90.

Der Röntgenkontrast-Stift 80 weist um den Umfang des Stifts verteilte, in Längsrichtung verlaufende Zähne 92 auf.

Die Vorpositionierungsstellung wird durch Einbringen des Stifts 80 zwischen den sich verjüngenden Bereich 88 und den Vorsprung 90 der Bohrung beim Hersteller eingestellt. Dabei ist der Röntgenkontrast-Stift 80 axial mit einem Spiel von 0,2 mm gehalten. Die Zähne 92 halten den Stift innerhalb des Bereichs 87 mit dem großen Durchmesser radial zentriert mit einem Spiel von 0,2 mm. Die Berührungsstellen der Spitzen der Zähne 92 mit der Innenwand der Bohrung 82 sind linienförmig. Dadurch werden alle Oberflächen im Inneren der Bohrung 82 wirksam mit Gas sterilisiert.

Die in Fig. 5c dargestellte Endpositionierungsstellung wird durch Drücken des Stifts 80 von der Vorpositionierungsstellung in Richtung des geschlossenen Endes 86 der Bohrung eingestellt. Dabei ist der Stift im Bereich 89 am Ende 86 der Bohrung kraft- und formschlüssig eingeklemmt. Die Zähne 92 greifen in die Innenwand der Bohrung mit kleinerem Durchmesser ein und fixieren auf diese Weise den Stift 80 in der Bohrung 82.

### Sechste Ausführungsform

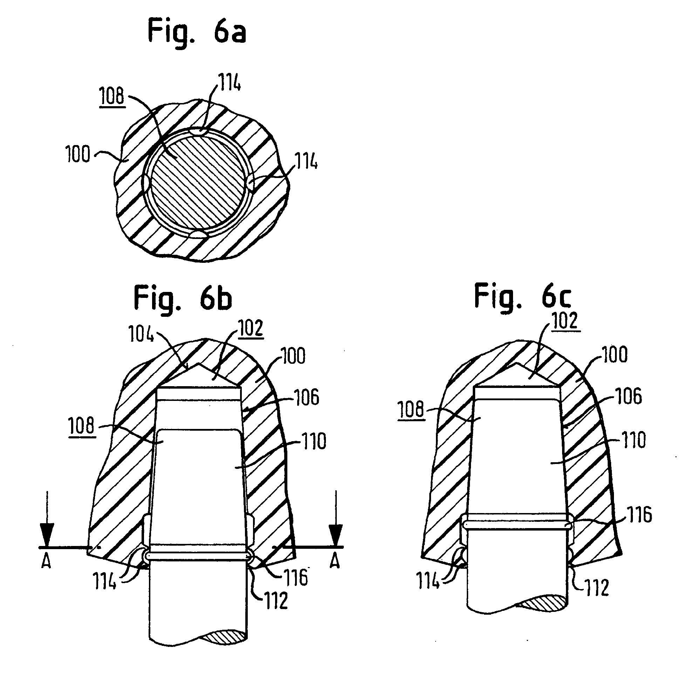

Die in Fig. 6a bis 6c gezeigte sechste Ausführungsform umfasst einen Stift 108 aus Metall und ein Element 100 aus UHMWPE. Der Stift 108 dient als mechanisches Führungselement zu benachbarten Implantatelementen. Beispielsweise ist der Stift 108 als Bolzen in einer beweglichen Tibia-Einlage (gliding meniscus) aus hochvernetztem UHMWPE zur Führung gegenüber einer Tibia-Metallverstärkung verankert.

Der Stift 108 besitzt ein konisches Ende 110 und einen ringförmigen Vorsprung 116, der angrenzend an das konische Ende 110 angeordnet ist.

Das Element 100 enthält eine Bohrung 102 mit einem zum konischen Ende 110 des Stifts komplementären Bereich 106. Die Bohrung 102 weist ferner an ihrem offenen Ende 112 Vorsprünge 114 auf, die in Form von zwei um den Umfang der Bohrung parallel zueinander angeordneten Ringen vorgesehenen sind.

In der Vorpositionierungsstellung ist der Vorsprung 116 zwischen den zwei Ringen der Vorsprünge 114 axial und radial mit Spiel gehalten. Dabei ist das konische Ende 110 des Stifts ebenfalls mit einem Spiel im Bereich 106 der Bohrung gelagert. Die Kontaktstellen zwischen den Vorsprüngen 114 und dem Stift 108 sind punktförmig, wie aus Fig. 6a erkennbar ist.

Das radiale und axiale Spiel und die punktförmigen Kontaktstellen gewährleisten, dass bei der Sterilisation praktisch alle Oberflächen innerhalb der Bohrung 102 vom Sterilisationsgas umströmt werden.

Eine in Fig. 6c dargestellte Endpositionierungsstellung des Stifts 108 wird ausgehend von der Vorpositionierungsstellung durch Drücken des Stifts 108 in Richtung des geschlossenen Endes 104 der Bohrung eingestellt. Dabei wird das konische Ende 110 des Stifts in dem dazu komplementären Bereich 106 der Bohrung geklemmt. Der Vorsprung 116 des Stifts 108 befindet sich nun in einem Querschnittsbereich der Bohrung 102, der zwischen den Vorsprüngen 114 und dem konisch sich verjüngenden Bereich 106 vorgesehen ist. Der freie Weg des Vorsprungs 116 in Längsrichtung der Bohrung stellt sicher, dass die Konusverankerung beim Einstellen der Endpositionierungsstellung nicht behindert wird.

### Siebte Ausführungsform

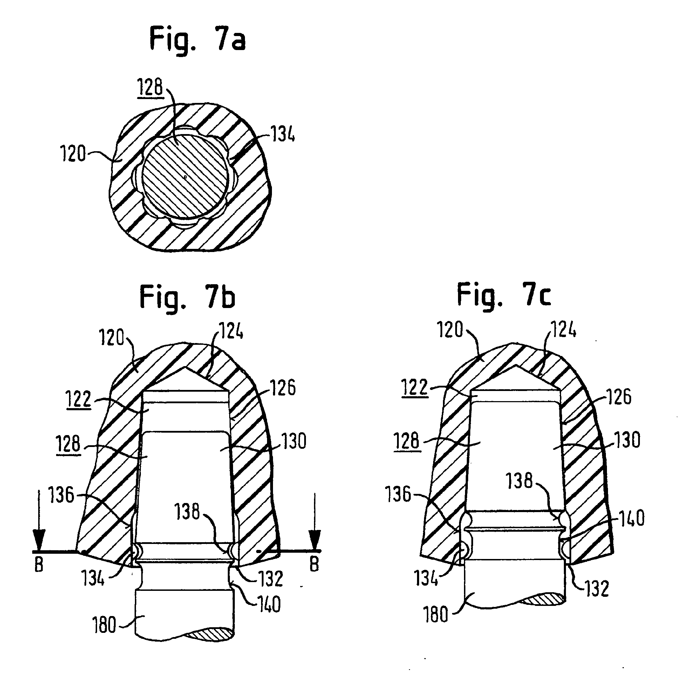

Die in Fig. 7a bis 7c gezeigte siebte Ausführungsform umfasst einen Stift 128 aus Metall und ein Element 120 aus UHMWPE.

Der Stift 128 besitzt ein konisches Ende 130. Der Stift 128 weist ferner angrenzend an das konische Ende 130 zwei hintereinander angeordnete Vertiefungen 138 und 140 auf, die jeweils ringförmig in den Stift 128 eingebracht sind. Die unmittelbar hinter dem konischen Ende 130 des Stifts angeordnete ringförmige Vertiefung 138 besitzt eine an Vorsprünge 134 am Element 120 angepasste Kontur, während die Vertiefung 140 in Längsrichtung des Stifts 128 im Vergleich zu der Vertiefung 138 größer ausgebildet ist.

Im Element 120 ist eine Bohrung 122 vorgesehen, die einen zum konischen Ende 130 des Stifts komplementären Bereich 126 aufweist. Die Bohrung 122 weist am offenen Ende 132 die Vorsprünge 134 auf, die in einem Ring um den Umfang der Bohrung herum verteilt sind. Zwischen dem Vorsprung 134 und dem konisch sich verjüngenden Bereich 126 liegt ein Bereich 136 der Bohrung, dessen Querschnitt größer ist als derjenige des Bereichs 126.

Die Vorpositionierungsstellung wird beim Hersteller vor der Gassterilisation durch Einschieben des Stifts 128 in die Bohrung 122 eingestellt.

Fig. 7a und 7b zeigen den Stift 128 in der Vorpositionierungsstellung, in der der Stift 128 durch In-Eingriff-Bringen der Vorsprünge 134 mit den Vertiefungen 138 mit einem radialen und axialen Spiel von beispielsweise 0,2 mm lose gehalten ist. Dabei ist das konische Ende 130 des Stifts innerhalb des konisch sich verjüngenden Bereichs 126 der Bohrung ebenfalls lose aufgenommen. Aus Fig. 7a ist ersichtlich, dass zwischen den Vorsprüngen 134 und dem Stift 128 ausschließlich punktförmige, spielbehaftete Berührungsstellen existieren.

Das Spiel zwischen den Oberflächen des Stifts 128 und den Innenwänden der Bohrung 122 stellt sicher, dass praktisch alle Oberflächen innerhalb der Bohrung 122 ausreichend vom Sterilisationsgas umströmt werden. In der Vorpositionierungsstellung erfolgt auch der sterile Transport des Implantats.

Nach dem Öffnen der Verpackung und kurz vor dem Einsetzen des Implantats 120 wird der Stift 128 ausgehend von der Vorpositionierungsstellung durch Pressen des Stifts in die Bohrung in der in Fig. 7c gezeigten Endpositionierungsstellung fixiert. Dabei sitzt das konische Ende 130 des Stifts in Klemmlage im Bereich 126 der Bohrung kraft- und formschlüssig fest.

### Achte Ausführungsform

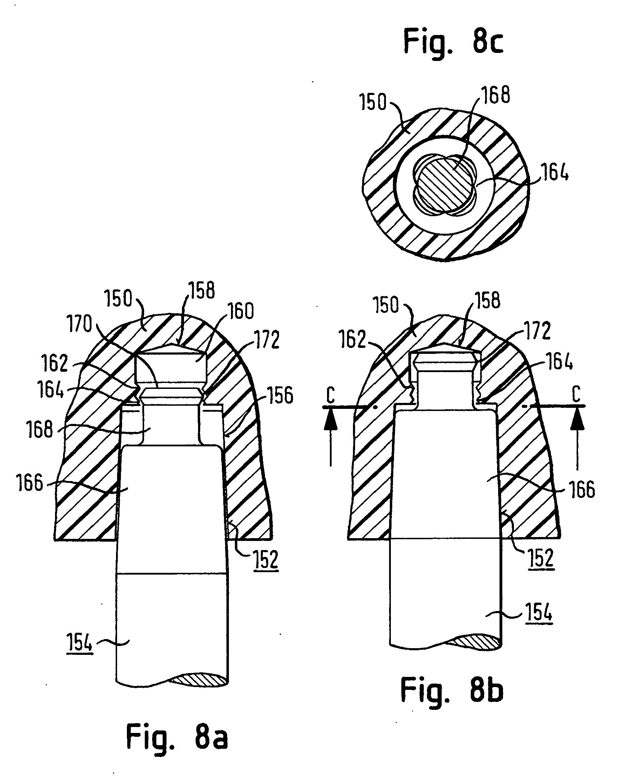

Fig. 8a bis 8c zeigen das erfindungsgemäße Implantat ausgeführt als Konus-Verbindung zwischen einem Implantat 150 mit einer Bohrung 152 und einem Stift 154. Das Implantat 150 besteht aus hochvernetztem ultrahochmolekularem Polyethylen, während es sich bei dem Stift 154 um einen Metallstift handelt.

Der Stift 154 weist einen konischen Bereich 166 auf, an den sich ein Zapfen 168 anschließt. Der Durchmesser des Zapfens ist kleiner als der Durchmesser des konischen Bereichs 166. Am flachen, vorderen Ende 170 des Zapfens 168 ist um den Umfang herum ein ringförmiger Vorsprung 172 ausgebildet.

Die Bohrung 152 weist einen zum konischen Bereich 166 des Stiftes 154 komplementären konischen Bereich 156 auf. Zwischen diesem und dem geschlossenen Ende 158 der Bohrung 152 ist ein Bereich 160 mit einem im Vergleich zum konischen Bereich 156 kleineren Durchmesser vorgesehen. Der Bereich 160 weist an seinem Eingang ins Innere der Bohrung spitz zulaufende Vorsprünge 162 und 164 auf, die zwei axial aufeinanderfolgende Ringteile bilden.

Die in Fig. 8a dargestellte Vorpositionierungsstellung wird durch Einbringen des Stifts 154 in die Bohrung 152 und Einschnappen des Vorsprungs 172 in den Zwischenraum zwischen den Vorsprüngen 162 und 164 eingestellt. Dabei ist der Vorsprung 172 des Stifts 154 zwischen den Vorsprüngen 162 und 164 mit einem radialen und axialen Spiel gehalten. Der konische Bereich 166 des Stiftes ist im konischen Bereich 156 der Bohrung mit einem Spiel lose gehalten. Die spielbehafteten Kontaktstellen zwischen dem Stift 154 und den Vorsprüngen 162, 164 sind aufgrund der Form der Vorsprünge punktförmig.

So wird gewährleistet, dass das Sterilisationsgas auf praktisch alle Oberflächen innerhalb der Bohrung 152 einwirken kann.

Die in Fig. 8b und 8c dargestellte Endpositionierungsstellung wird kurz vor dem Implantieren ausgehend von der Vorpositionierungsstellung durch Drücken des Stifts 154 in Richtung des geschlossenen Endes der Bohrung 152 eingestellt. Dabei sitzt der Stift 154 mit seinem konischen Bereich 166 in der Bohrung kraft- und formschlüssig fest.

### Neunte Ausführungsform

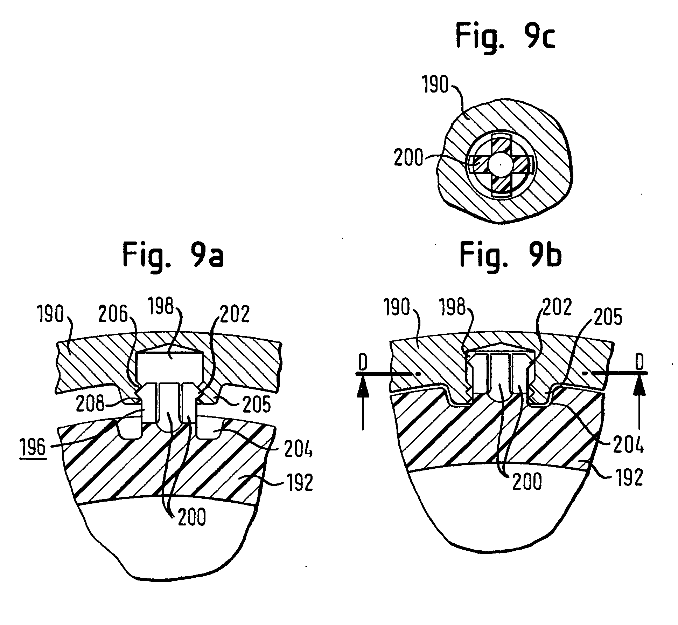

Die in Fig. 9a bis 9c gezeigte neunte Ausführungsform betrifft ein Implantat bestehend aus einer Außenschale 190 und einer Innenschale 192, beispielsweise für eine Hüftgelenksprothese. Die Außenschale 190 besteht aus einem Metall, beispielsweise einer Titanlegierung, und die Innenschale ist aus hochvernetztem ultrahochmolekularem Polyethylen gebildet. Diese ist in der Außenschale 190 mittels einer Schnappfeder 196 der Innenschale und einer Ausnehmung 198 in der Außenschale 190 in einer Vorpositionierungsstellung zentriert gehalten.

Die Schnappfeder 196 der Innenschale besteht aus vier, um 90° versetzt im Kreis angeordneten federnden Stegen 200. An der Außenseite der Stege 200 ist am freien Ende der Stege jeweils ein Vorsprung 202 vorgesehen.

Die Ausnehmung 198 weist an ihrem offenen Ende um den Umfang der Ausnehmung 198 herum zwei parallel zueinander angeordnete, ringförmige Vorsprünge 206 und 208 auf.

Die in Fig. 9a gezeigte Vorpositionierungsstellung wird vor der Gassterilisation des Implantats eingestellt. Dabei sind die Stege 200 in einer Schnappverbindung mit ihren Vorsprüngen 202 zwischen den Vorsprüngen 206 und 208 der Ausnehmung gehalten. Die Kontaktstellen sind kleinflächig und haben ein radiales und axiales Spiel. Zwischen den anderen Oberflächen der Außenschale 190 und der Innenschale 192 ist ein im Vergleich zu diesem Spiel von beispielsweise 0,2 mm großer Zwischenraum vorhanden. Dieser und das Spiel der Schnappverbindung zwischen den Vorsprüngen 202, 206 und 208, sowie die Zwischenräume zwischen den Stegen 200 gewährleisten, dass praktisch alle Oberflächen innerhalb der Ausnehmung 198 von dem Sterilisiergas beim Sterilisieren erreicht werden können.

Die Endpositionierungsstellung wird durch Verlagern der Innenschale 192 von der Vorpositionierungsstellung in Richtung der Außenschale 190 realisiert, wobei der Vorsprung 202 der Schnappfeder über den Vorsprung 206 gedrückt wird. Wie in Fig. 9b und 9c gezeigt, ist die Schnappfeder 196 dabei fast vollständig in die Ausnehmung 198 eingeführt und zentriert die Innenschale 192 in der Außenschale 190, wobei der Vorsprung 206 der Außenschale 190 formschlüssig in eine korrespondierende Ausnehmung 204 der Innenschale 192 eingreift. Die eigentliche Verankerung zwischen Außen- und Innenschale findet durch eine Schnappverbindung am Äquator statt.

### Zehnte Ausführungsform

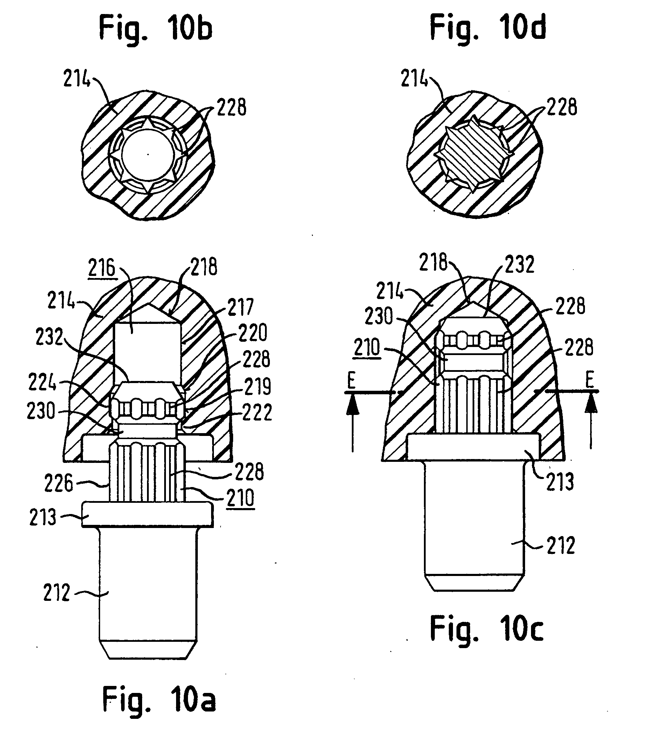

Die zehnte, in Fig. 10a bis 10d gezeigte Ausführungsform des erfindungsgemäßen Implantats betrifft eine Zapfen-Verankerung. Sie umfaßt einen Stift 210 aus Metall, der ein Verankerungsteil 212 und einen Flansch 213 aufweist, sowie ein Implantat 214 mit einer Bohrung 216.

Die Bohrung weist vor dem geschlossenen Ende 218 einen Bereich 217 mit einem kleinen Durchmesser und zwischen diesem und dem offenen Ende einen Bereich 219 mit größerem Durchmesser auf. Zwischen den Bereichen 217 und 219 ist ein sich zum geschlossenen Ende 218 der Bohrung hin verjüngender Bereich 220 vorgesehen. Mit Abstand zu dem sich verjüngenden Bereich 220 ist im Eintrittsbereich 219 der Bohrung ein um die Bohrung umlaufender Vorsprung 222 vorgesehen.

Der Stift 210 weist zwei Abschnitte 224 und 226 auf, die mit in Längsrichtung des Stifts verlaufenden Zähnen 228 versehen sind. In den Abschnitten 224 und 226 besitzt der Stift somit einen in Fig. 10b dargestellten sternförmigen Querschnitt. Die Abschnitte 224 und 226 sind durch einen nicht mit Zähnen versehenen Abschnitt 230 getrennt, der einen kleineren Querschnitt als die Abschnitte 224 und 226 aufweist.

Die in Fig. 10a und 10b dargestellte Vorpositionierungsstellung wird vor der Durchführung einer Gassterilisation, insbesondere einer Sterilisation mit Ethylenoxid, eingestellt. Dabei ist der Abschnitt 224 des Stifts zwischen dem sich verjüngenden Bereich 220 und dem Vorsprung 222 der Ausnehmung angeordnet. In dieser Position existiert aufgrund der Zähne 228, die den Stift innerhalb der Ausnehmung zentriert halten, zwischen dem Stift und der Innenwand der Ausnehmung ein radiales Spiel. Zudem ist ein axiales Spiel vorhanden. Die Berührungsstellen sind in der Vorpositionierungsstellung zwischen den Zähnen 228 und dem Implantat 214 spielbehaftet punktförmig. Somit sind praktisch alle Oberflächen innerhalb der Bohrung 216 bei der Gassterilisation vom Gas erreichbar.

Die in Fig. 10c und 10d dargestellte Endpositionierungsstellung wird ausgehend von der Vorpositionierungsstellung kurz vor dem Einsetzen des Implantats durch Pressen des Stifts 210 in die Bohrung 216 und Eingraben der Zähne 228 in die Innenwand der Bohrung 216 erreicht. Auf diese Weise ist der Stift 210 drehfest in der Bohrung 216 verankert. Der Flansch 213 des Verankerungsteils 212 schließt dabei, in einer Ausnehmung angeordnet, mit dem offenen Ende der Bohrung 216 bündig ab, so dass nur das Verankerungsteil 212 aus dem Implantat 214 herausragt.

Das Verankerungsteil 212 kann als drehfeste Basis für andere Elemente des Implantats verwendet werden. Das Verankerungsteil 212 kann dabei in Bezug auf den Stift 210 rotationssymmetrisch, als Exzenterzapfen oder als gegabelte Aufnahme ausgebildet sein. Die Gabelung der Aufnahme wird bei der Einstellung der Endpositionierung intraoperativ wie benötigt ausgerichtet.

### Bezugszeichenliste

- 2: Hüftgelenksprothesenschale
- 4: Außenschale
- 6: Innenschale
- 8: Abdeckblech
- 10: Öffnungen
- 12: Ausnehmung
- 14: Vorsprünge
- 16: Bohrung
- 18: Schnappverbindung
- 20: Schrägboden
- 22: Seitenwand
- 24: Vorsprung
- 26: Rille
- 28: Nocke
- 30: Nut
- 32: Einschubführung
- 40: Röntgenkontrast-Kugel
- 42: Bohrung
- 44: Element
- 46: Vorsprung
- 47: Bereich
- 48: Vorsprung
- 49: Bereich
- 50: geschlossenes Ende
- 52: Stopfen
- 60: Röntgenkontrast-Kugel
- 61: Schnappverbindung
- 62: Körbchen
- 63: Element
- 64: Aufnahme
- 66: Bohrung
- 66: Arm
- 70: Vorsprung
- 72: Vertiefung
- 74: geschlossenes Ende
- 76: Vertiefung
- 80: Röntgenkontrast-Stift
- 82: Bohrung
- 84: Element
- 86: geschlossenes Ende
- 87: Bereich
- 88: sich verjüngender Bereich
- 89: Bereich
- 90: Vorsprung
- 92: Zahn
- 94: sich verjüngender Bereich
- 100: Element
- 102: Bohrung
- 104: geschlossenes Ende
- 106: Bereich
- 108: Stift
- 110: konisches Ende
- 112: offenes Ende
- 114: Vorsprung
- 116: Vorsprung
- 120: Element
- 122: Bohrung
- 124: geschlossenes Ende
- 126: Bereich
- 128: Stift
- 130: konisches Ende
- 132: offenes Ende
- 134: Vorsprung
- 136: Bereich
- 138: Vertiefung
- 140: Vertiefung
- 150: Element
- 152: Bohrung
- 154: Metallstift
- 156: Bereich
- 158: geschlossenes Ende
- 160: Bereich
- 162: Vorsprung
- 164: Vorsprung
- 166: konischer Bereich
- 168: Zapfen
- 170: vorderes Ende
- 172: Vorsprung
- 190: Außenschale
- 192: Innenschale
- 196: Schnappfeder
- 198: Ausnehmung
- 200: Steg
- 202: Vorsprung
- 204: Ausnehmung
- 206: Vorsprung
- 208: Vorsprung
- 210: Stift
- 212: Verankerungsteil
- 213: Flansch
- 214: Element
- 216: Bohrung
- 217: Bereich
- 218: geschlossenes Ende
- 219: Bereich
- 220: sich verjüngender Bereich
- 222: Vorsprung
- 224: Abschnitt
- 226: Abschnitt
- 228: Zahn
- 230: Abschnitt
- 232: vorderes Ende

## Patentansprüche

1. Implantat mit
wenigstens einem ersten Element (8; 40; 60, 62; 80; 108; 128; 154; 192; 210) und einem zweiten Element (6; 44; 63; 84; 100; 120; 150; 190; 214), wobei das erste Element in einer vorgegebenen Relativlage zum zweiten Element vorgesehen ist,
**dadurch gekennzeichnet, dass**
das erste Element (8; 40; 60, 62; 80; 108; 128; 154; 192; 210) mit dem zweiten Element (6; 44; 63; 84; 100; 120; 150; 190; 214) in einer spielbehafteten, eine vollflächige Gassterilisation beider Elemente gewährleistenden Vorpositionierungsstellung mechanisch verbunden ist und aus dieser Vorpositionierungsstellung in eine einem Festverbund beider Elemente entsprechende Endpositionierungsstellung verlagerbar ist.

2. Implantat nach Anspruch 1,
**dadurch gekennzeichnet, dass**
das erste Element (8; 40; 60, 62; 80; 108; 128; 154; 192; 210) zumindest teilweise in einer Ausnehmung (12; 42; 66; 82; 102; 122; 152; 198; 216) des zweiten Elements (6; 44; 63; 84; 100; 120; 150; 190; 214) vorgesehen ist,
wobei das erste Element (8; 40; 60, 62; 80; 108; 128; 154; 192; 210) in der Vorpositionierungsstellung in der Ausnehmung (12; 42; 66; 82; 102; 122; 152; 198; 216) lose gehalten ist, wobei die Berührungsstellen zwischen dem ersten Element und dem zweiten Element in der Ausnehmung kleinflächig sind, und
in der Endpositionierungsstellung in der Ausnehmung mit dem zweiten Element (6; 44; 63; 84; 100; 120; 150; 190; 214) kraft- und formschlüssig verbunden ist.

3. Implantat nach Anspruch 1 oder 2,
**dadurch gekennzeichnet, dass**
das Material mindestens eines der ersten und zweiten Elemente (6, 8; 40, 44; 60, 62, 63; 80, 84; 100, 108; 120, 128; 150, 154; 190, 192; 210, 214) hochvernetztes ultrahochmolekulares Polyethylen und/oder einen anderen Kunststoff umfasst.

4. Implantat nach einem der vorstehenden Ansprüche,
**dadurch gekennzeichnet, dass**
das Material eines der ersten und zweiten Elemente (6, 8; 40, 44; 60, 62, 63; 80, 84; 100, 108; 120, 128; 150, 154; 190, 192; 210, 214) ein Metall, insbesondere Titan, umfasst.

5. Implantat nach einem der vorstehenden Ansprüche,
**dadurch gekennzeichnet, dass**
das erste Element (8; 40; 60, 62; 80; 108; 128; 154; 192; 210) in der Vorpositionierungsstellung im wesentlichen zentriert lose gehalten ist.

6. Implantat nach einem der vorstehenden Ansprüche,
**dadurch gekennzeichnet, dass**
das erste Element (8; 40; 60, 62; 80; 108; 128; 154; 192; 210) in der Vorpositionierungsstellung mit einem Spiel von mindestens 0,1 mm gehalten ist.

7. Implantat nach einem der Ansprüche 2 bis 6,
**dadurch gekennzeichnet, dass**
die Ausnehmung einen im wesentlichen kreisförmigen Querschnitt aufweist, das erste Element (8; 40; 60, 62; 80; 108; 128; 154; 192; 210) einen im wesentlichen kreisförmigen und/oder im wesentlichen sternförmigen Querschnitt aufweist, und das erste Element in der Vorpositionierungsstellung in der Ausnehmung (12; 42; 66; 82; 102; 122; 152; 198; 216) radial und axial lose gehalten ist.

8. Implantat nach einem der Ansprüche 2 bis 7,
**dadurch gekennzeichnet, dass**
in der Vorpositionierungsstellung die Berührungsstellen zwischen dem ersten Element (8; 40; 60, 62; 80; 108; 128; 154; 192; 210) und dem zweiten Element (6; 44; 63; 84; 100; 120; 150; 190; 214) in der Ausnehmung (12; 42; 66; 82; 102; 122; 152; 198; 216) im wesentlichen punktförmig sind.

9. Implantat nach einem der Ansprüche 2 bis 8,
**dadurch gekennzeichnet, dass**
in der Vorpositionierungsstellung die Anzahl der Berührungsstellen in der Ausnehmung zwischen dem ersten Element (8; 40; 60, 62; 80; 108; 128; 154; 192; 210) und dem zweiten Element (6; 44; 63; 84; 100; 120; 150; 190; 214) gering gehalten ist.

10. Implantat nach einem der vorstehenden Ansprüche,
**dadurch gekennzeichnet, dass**
das erste Element (40; 60, 62; 80; 108; 128; 154; 192; 210) in der Vorpositionierungsstellung mittels einer Schnappverbindung lose gehalten ist.

11. Implantat nach einem der Ansprüche 2 bis 10,
**dadurch gekennzeichnet, dass**
die Ausnehmung (82) mindestens einen Vorsprung (90) und einen sich am Ende der Ausnehmung verjüngenden Bereich (88) aufweist, und
das erste Element (80; 210) in der Vorpositionierungsstellung zwischen dem Vorsprung (90) und dem sich verjüngenden Bereich (88) lose gehalten ist.

12. Implantat nach einem der Ansprüche 2 bis 11,
**dadurch gekennzeichnet, dass**
die Ausnehmung mindestens zwei Vorsprünge (46, 48; 114; 162, 164; 206; 208) aufweist, und
das erste Element (40; 108; 154) in der Vorpositionierungsstellung zwischen mindestens zwei Vorsprüngen lose gehalten ist.

13. Implantat nach einem der Ansprüche 2 bis 12,
**dadurch gekennzeichnet, dass**
die Ausnehmung (122) mindesten einen Vorsprung (134) aufweist, das erste Element (128) mindestens zwei Vertiefungen (138; 140) zur Aufnahme des Vorsprungs (134) aufweist,
das erste Element (128) in der Vorpositionierungsstellung durch den in eine der mindestens zwei Vertiefungen (138) eingreifenden Vorsprung (134) lose gehalten ist, und
der Vorsprung (134) in der Endpositionierungsstellung in einer anderen der mindestens zwei Vertiefungen (140) positioniert ist.

14. Implantat nach einem der vorstehenden Ansprüche,
**dadurch gekennzeichnet, dass**
das erste Element (40; 60, 62; 80; 108; 128; 154; 192; 210) in der Endpositionierungsstellung mit dem zweiten Element (6; 44; 63; 84; 100; 120; 150; 190; 214) im Klemmsitz und/oder mittels einer Schnappverbindung kraft- und formschlüssig verbunden ist.

15. Implantat nach einem der vorstehenden Ansprüche,
**dadurch gekennzeichnet, dass**
das erste Element (8; 80; 210) mindestens einen Vorsprung aufweist, der in der Endpositionierungsstellung in das zweite Element (6; 84; 214) eingreift.

16. Implantat nach einem der vorstehenden Ansprüche,
**dadurch gekennzeichnet, dass**
das erste Element (40; 60; 80; 108; 128; 154; 210) eine Kugel (40; 60) oder ein Stift (80; 108; 128; 154; 210), insbesondere eine Metallkugel oder ein Metallstift, ist.

17. Implantat nach einem der Ansprüche 2 bis 16,
**dadurch gekennzeichnet, dass**
das erste Element (60, 62) eine Kugel (60) und einen Stopfen (62) mit einer Aufnahme für die Kugel umfasst,
in der Vorpositionierungsstellung die Kugel (60) in dem Stopfen (62) oder zwischen dem Stopfen (62) und dem Ende der Ausnehmung lose gehalten ist, und
in der Endpositionierungsstellung der Stopfen (62) in der Ausnehmung und die Kugel (60) in dem Stopfen (62) kraft- und formschlüssig gehalten ist.

18. Implantat nach einem der vorstehenden Ansprüche,
**dadurch gekennzeichnet, dass**
das erste Element ein Stift (210) mit einem Verankerungs-Zapfen (212) ist.

19. Implantat nach einem der vorstehenden Ansprüche,
**dadurch gekennzeichnet, dass**
das zweite Element eine Innenschale (6) einer Gelenkprothese und das erste Element eine Abdeckscheibe (8) der Innenschale ist, eine Ausnehmung (12) in der Außenwand der Innenschale mit einem kreisförmigen Querschnitt, einer Seitenwand (22) und einem zum Zentrum der Ausnehmung tiefer werdenden Boden (20) vorgesehen ist,
wobei die Seitenwand (22) mindestens einen zum Zentrum der Ausnehmung gerichteten Vorsprung (24) aufweist,
die Abdeckscheibe (8) in der Vorpositionierungsstellung von der Seitenwand und von dem Vorsprung lose gehalten ist,
die Abdeckscheibe (8) mindestens einen Nocken (28) aufweist, der in der Endpositionierungsstellung in die Seitenwand (22) der Ausnehmung eingreift, und
die Abdeckscheibe (8) in der Ausnehmung (12) durch eine Drehbewegung von der Vorpositionierungsstellung in die Endpositionierungsstellung verlagerbar ist.

20. Implantat nach Anspruch 19,
**dadurch gekennzeichnet, dass**
in der Seitenwand (22) der Ausnehmung mindestens eine Aufnahme (30) für den Nocken (28) in der Vorpositionierungsstellung vorgesehen ist.

21. Implantat nach Anspruch 19 oder 20,
**dadurch gekennzeichnet, dass**
die Ausnehmung (12) im Übergang von der Seitenwand (22) zum Boden (20) eine um die Ausnehmung verlaufende Rille (26) aufweist, und/oder dass die Seitenwand (22) der Ausnehmung eine Einschubführung (32) zum Einbringen des ersten Elements (8) in die Vorpositionierungsstellung aufweist.

22. Anordnung aus einem Implantat nach einem der vorstehenden Ansprüche und einer Verpackung, in welcher das Implantat gassterilisiert verpackt ist,
**dadurch gekennzeichnet, dass**
die Elemente (6, 8; 40, 44; 60, 62, 63; 80, 84; 100, 108; 120, 128; 150, 154; 190, 192; 210, 214) in der gasdurchlässigen und für Mikroorganismen undurchlässigen Verpackung die Vorpositionierungsstellung einnehmen, aus der sie nach Öffnen der Verpackung durch Dreh- und/oder Schiebebewegungen in die Endpositionierungsstellung überführbar sind.

23. Verfahren zum Bereitstellen einer Anordnung aus einem Implantat nach einem der Ansprüche 1 bis 21 und einer Verpackung, in der das Implantat sterilisiert verpackt und mit der das Implantat transportierbar und lagerbar ist,
bei dem die das Implantat bildenden Elemente (6, 8; 40, 44; 60, 62, 63; 80, 84; 100, 108; 120, 128; 150, 154; 190, 192; 210, 214) zunächst in die Vorpositionierungsstellung gebracht, dann in der Vorpositionierungsstellung in die Verpackung eingebracht und anschließend mit Gas sterilisiert werden.

24. Verfahren nach Anspruch 23,
**dadurch gekennzeichnet, dass**
die das Implantat bildenden Elemente (6, 8; 40, 44; 60, 62, 63; 80, 84; 100, 108; 120, 128; 150, 154; 190, 192; 210, 214) bei Transport und Lagerung in der Verpackung in der Vorpositionierungsstellung verbleiben.

25. Verfahren nach Anspruch 23 oder 24,
**dadurch gekennzeichnet, dass**
die Gassterilisation mit Ethylenoxid durchgeführt wird.

26. Verfahren nach einem der Ansprüche 23 bis 25,
**dadurch gekennzeichnet, dass**
die Verpackung nach dem Einbringen der Elemente (6, 8; 40, 44; 60, 62, 63; 80, 84; 100, 108; 120, 128; 150, 154; 190, 192; 210, 214) und vor der Gassterilisation mit einer gasdurchlässigen und für Mikroorganismen undurchlässigen Membran verschlossen wird.

## Claims

1. An implant comprising
at least one first element (8; 40; 60, 62; 80; 108; 128; 154; 192; 210) and a second element (6; 44; 63; 84; 100; 120; 150; 190; 214), wherein the first element is provided in a predefined relative position to the second element,
**characterized in that**
the first element (8; 40; 60, 62; 80; 108; 128; 154; 192; 210) is mechanically coupled to the second element (6; 44; 63; 84; 100; 120; 150; 190; 214) in a pre-positioning location having play and ensuring a full area gas sterilzation of both elements and can be displaced from this pre-positioning location into an end positioning location corresponding to a fixed assembly of both elements.

2. An implant in accordance with claim 1,
**characterised in that**
the first element (8; 40; 60, 62; 80; 108; 128; 154; 192; 210) is provided at least in part in a recess (12; 42; 66; 82; 102; 122; 152; 198; 216) of the second element (6; 44; 63; 84; 100; 120; 150; 190; 214),
wherein the first element (8; 40; 60, 62; 80; 108; 128; 154; 192; 210) is loosely held in the pre-positioning location in the recess (12; 42; 66; 82; 102; 122; 152; 198; 216), wherein the contact points between the first element and the second element in the recess are small in area; and
is connected in a force locked and shape matched manner to the second element (6; 44; 63; 84; 100; 120; 150; 190; 214) in the end positioning location in the recess.

3. An implant in accordance with claim 1 or claim 2,
**characterised in that**
the material of at least one of the first and second elements (6, 8; 40, 44; 60, 62, 63; 80, 84; 100, 108; 120, 128; 150, 154; 190, 192; 210, 214) includes highly cross-linked ultra-high molecular weight polyethylene and/or another plastic.

4. An implant in accordance with any one of the preceding claims,
**characterized in that**
the material of one of the first and second elements (6, 8; 40, 44; 60, 62, 63; 80, 84; 100, 108; 120, 128; 150, 154; 190, 192; 210, 214) includes a metal, in particular titanium.

5. An implant in accordance with any one of the preceding claims,
**characterized in that**
the first element (8; 40; 60, 62; 80; 108; 128; 154; 192; 210) is loosely held in a substantially centred manner in the pre-positioning location.

6. An implant in accordance with any one of the preceding claims,
**characterized in that**
the first element (8; 40; 60, 62; 80; 108; 128; 154; 192; 210) is held in the pre-positioning location with a play of at least 0.1 mm.

7. An implant in accordance with any one of the claims 2 to 6,
**characterized in that**
the recess has a substantially circular cross-section, the first element (8; 40; 60, 62; 80; 108; 128; 154; 192; 210) has a substantially circular and/or substantially star-shaped cross-section and the first element is radially and axially loosely held in the pre-positioning location in the recess (12; 42; 66; 82; 102; 122; 152; 198; 216).

8. An implant in accordance with any one of the claims 2 to 7,
**characterized in that**,
in the pre-positioning location, the contact points between the first element (8; 40; 60, 62; 80; 108; 128; 154; 192; 210) and the second element (6; 44; 63; 84; 100; 120; 150; 190; 214) in the recess (12; 42; 66; 82; 102; 122; 152; 198; 216) are substantially punctiform.

9. An implant in accordance with any one of the claims 2 to 8,
**characterized in that**,
in the pre-positioning location, the number of contact points in the recess between the first element (8; 40; 60, 62; 80; 108; 128; 154; 192; 210) and the second element (6; 44; 63; 84; 100; 120; 150; 190; 214) is kept low.

10. An implant in accordance with any one of the preceding claims,
**characterized in that**
the first element (40; 60, 62; 80; 108; 128; 154; 192; 210) is loosely held in the pre-positioning location by means of a snap-in connection.

11. An implant in accordance with any one of the claims 2 to 10,
**characterized in that**
the recess (82) has at least one projection (90) and a tapering region (88) at the end of the recess; and
the first element (80; 210) is loosely held in the pre-positioning location between the projection (90) and the tapering region (88).

12. An implant in accordance with any one of the claims 2 to 11,
**characterized in that**
the recess has at least two projections (46, 48; 114; 162, 164; 206; 208); and
the first element (40; 108; 154) is loosely held in the pre-positioning location between at least two projections.

13. An implant in accordance with any one of the claims 2 to 12,
**characterized in that**
the recess (122) has at least one projection (134);
the first element (128) has at least two depressions (138; 140) for receiving the projection (134);
the first element (12) is loosely held in the pre-positioning location by the projection (134) engaging into one of the at least two depressions (138); and
the projection (134) is positioned in the end positioning location in another of the at least two depressions (140).

14. An implant in accordance with any one of the preceding claims,
**characterized in that**
the first element (40; 60, 62; 80; 108; 128; 154; 192; 210) is connected in a force locked and shape matched manner in the end positioning location to the second element (6; 44; 63; 84; 100; 120; 150; 190; 214) in a clamping fit and/or by means of a snap-in connection.

15. An implant in accordance with any one of the preceding claims,
**characterized in that**
the first element (8; 80; 210) has at least one projection which engages into the second element (6; 84; 214) in the end positioning location.

16. An implant in accordance with any one of the preceding claims,
**characterized in that**
the first element (40; 60; 80; 108; 128; 154; 210) is a ball (40; 60) or a pin (80; 108; 128; 154; 210), in particular a metal ball or a metal pin.

17. An implant in accordance with any one of the claims 2 to 16,
**characterized in that**
the first element (60, 62) includes a ball (60) and a plug (62) having a receiver for the ball;
the ball (60) is loosely held in the pre-positioning location in the plug (62) or between the plug (62) and the end of the recess; and, in the end positioning location, the plug (62) is held in the recess and the ball (60) is held in the plug (62) in a force locked and shape matched manner.

18. An implant in accordance with any one of the preceding claims,
**characterized in that**
the first element is a pin (210) having an anchoring spigot (212).

19. An implant in accordance with any one of the preceding claims,
**characterized in that**
the second element is an inner shell (6) of a joint prosthesis and the first element is a cover plate (8) of the inner shell;
a recess (12) is provided in the outer wall of the inner shell with a circular cross-section, a side wall (22) and a base (20) becoming deeper towards the centre of the recess,
wherein the side wall (22) has at least one projection (24) directed to the centre of the recess;
the cover plate (8) is loosely held by the side wall and by the projection in the pre-positioning location;
the cover plate (8) has at least one cam (28) which engages, in the end positioning location, into the side wall (22) of the recess; and the cover plate (8) can be displaced in the recess (12) from the pre-positioning location into the end positioning location by a rotational movement.

20. An implant in accordance with claim 19,
**characterised in that**
at least one receiver (30) for the cam (28) is provided in the side wall (22) of the recess in the pre-positioning location.

21. An implant in accordance with claim 19 or claim 20,
**characterised in that**
the recess (12) has a groove (26) extending about the recess at the transition from the side wall (22) to the base (20);
and/or **in that** the side wall (22) of the recess has an insert guide (32) for the insertion of the first element (8) into the pre-positioning location.

22. An arrangement of an implant in accordance with any one of the preceding claims and of a packaging in which the implant is packed in a gas-sterilized manner,
**characterized in that**
the elements (6, 8; 40, 44; 60, 62, 63; 80, 84; 100, 108; 120, 128; 150, 154; 190, 192; 210, 214) in the gas-permeable packaging impermeable for microorganisms adopt the pre-positioning position from which they can be moved into the end positioning position after opening the packaging by rotational and/or sliding movements.

23. A method of providing an arrangement of an implant in accordance with any one of the claims 1 to 21 and of a packaging in which the impalnt is packed in a sterilized manner and with which the implant can be transported and stored:
wherein the elements (6, 8; 40, 44; 60, 62, 63; 80, 84; 100, 108; 120, 128; 150, 154; 190, 192; 210, 214) forming the implant are first brought into the pre-positoining location, are then introduced into the packaging in the pre-positoining location and are subsdquently sterilized with gas.

24. A method in accordance with claim 23,
**characterized in that**
the elements (6, 8; 40, 44; 60, 62, 63; 80, 84; 100, 108; 120, 128; 150, 154; 190, 192; 210, 214) forming the implant remain in the pre-positioning location during transport and storage in the packaging.

25. A method in accordance with claim 23 or claim 24,
**characterised in that**
the gas sterilzation is carried out with ethylene oxide.

26. A method in accordance with any one of the claims 23 to 25,
**characterised in that**
the packaging is closed by a gas-permeable membrane impermeable for microoranisms after the introduction of the elements (6, 8; 40, 44; 60, 62, 63; 80, 84; 100, 108; 120, 128; 150, 154; 190, 192; 210, 214) and before the gas sterilization.

## Revendications

1. Implant, comprenant
au moins un premier élément (8 ; 40 ; 60, 62 ; 80 ; 108 ; 128 ; 154 ; 192 ; 210) et un second élément (6 ; 44 ; 63 ; 84 ; 100 ; 120 ; ' 150 ; 190 ; 214), dans lequel le premier élément est prévu dans une position relative prédéterminée par rapport au second élément,
**caractérisé en ce que**
le premier élément (8 ; 40 ; 60, 62 ; 80 ; 108 ; 128 ; 154 ; 192 ; 210) est relié au second élément (6 ; 44 ; 63 ; 84 ; 100 ; 120 ; 150 ; 190 ; 214) de façon mécanique dans une situation de prépositionnement entachée de jeu et garantissant une stérilisation au gaz des deux éléments sur toute leur surface, et est déplaçable depuis cette situation de prépositionnement jusque dans une situation de positionnement final correspondant à une liaison solidaire des deux éléments.

2. Implant selon la revendication 1,
**caractérisé en ce que**
le premier élément (8 ; 40 ; 60, 62 ; 80 ; 108 ; 128 ; 154 ; 192 ; 210) est prévu au moins partiellement dans un évidement (12 ; 42 ; 66 ; 82 ; 102 ; 122 ; 152 ; 198 ; 216) du second élément (6 ; 44 ; 63 ; 84 ; 100 ; 120 ; 150 ; 190 ; 214),
dans lequel le premier élément (8 ; 40 ; 60, 62 ; 80 ; 108 ; 128 ; 154 ; 192 ; 210) est maintenu de manière lâche dans l'évidement (12 ; 42 ; 66; 82 ; 102 ; 122 ; 152 ; 198 ; 216) dans la situation de prépositionnement, et les emplacements de contact entre le premier élément le second élément dans l'évidement ont des petites surfaces, et dans la situation de positionnement final dans l'évidement, il est relié en coopération de forces et en coopération de formes avec le second élément (6 ; 44 ; 63 ; 84 ; 100 ; 120 ; 150 ; 190 ; 214).

3. Implant selon la revendication 1 ou 2,
**caractérisé en ce que**
le matériau de l'un au moins des premiers et des seconds éléments (6, 8 ; 40, 44 ; 60, 62, 63 ; 80, 84 ; 100, 108 ; 120, 128 ; 150, 154 ; 190, 192 ; 210, 214) inclut un polyéthylène fortement réticulé à poids moléculaire extrêmement élevé et/ou une autre matière plastique.

4. Implant selon l'une des revendications précédentes,
**caractérisé en ce que**
le matériau de l'un des premiers et des seconds éléments (6, 8 ; 40, 44 ; 60, 62, 63 ; 80, 84 ; 100, 108 ; 120, 128 ; 150, 154 ; 190, 192 ; 210, 214) inclut un métal, en particulier du titane.

5. Implant selon l'une des revendications précédentes,
**caractérisé en ce que**, dans la situation de prépositionnement, le premier élément (8 ; 40 ; 60, 62 ; 80 ; 108 ; 128 ; 154 ; 192 ; 210) est maintenu sensiblement centré et de manière lâche.

6. Implant selon l'une des revendications précédentes,
**caractérisé en ce que**, dans la situation de prépositionnement, le premier élément (8 ; 40 ; 60, 62 ; 80 ; 108 ; 128 ; 154 ; 192 ; 210) est maintenu avec un jeu d'au moins 0,1 mm.

7. Implant selon l'une des revendications 2 à 6,
**caractérisé en ce que**
l'évidement présente une section transversale de forme sensiblement circulaire, le premier élément (8 ; 40 ; 60, 62 ; 80 ; 108 ; 128 ; 154 ; 192 ; 210) présente une section transversale de forme sensiblement circulaire et/ou sensiblement en forme d'étoile et, dans la situation de prépositionnement, le premier élément est maintenu de manière lâche radialement et axialement dans l'évidement (12 ; 42 ; 66 ; 82 ; 102 ; 122 ; 152 ; 198 ; 216).

8. Implant selon l'une des revendications 2 à 7,
**caractérisé en ce que**,
dans la situation de prépositionnement, les emplacements de contact entre le premier élément (8 ; 40 ; 60, 62 ; 80 ; 108 ; 128 ; 154 ; 192 ; 210) et le second élément (6 ; 44 ; 63 ; 84 ; 100 ; 120 ; 150 ; 190 ; 214) dans l'évidement (12 ; 42 ; 66 ; 82 ; 102 ; 122 ; 152 ; 198 ; 216) sont sensiblement de forme ponctuelle.

9. Implant selon l'une des revendications 2 à 8,
**caractérisé en ce que**, dans la situation de prépositionnement, le nombre des emplacements de contact dans l'évidement entre le premier élément (8 ; 40 ; 60, 62 ; 80 ; 108 ; 128 ; 154 ; 192 ; 210) et le second élément (6 ; 44 ; 63 ; 84 ; 100 ; 120 ; 150 ; 190 ; 214) est maintenu faible.

10. Implant selon l'une des revendications précédentes,
**caractérisé en ce que**, dans la situation de prépositionnement, le premier élément (8 ; 40 ; 60, 62 ; 80 ; 108 ; 128 ; 154 ; 192 ; 210) est maintenu de manière lâche au moyen d'une liaison à encliquetage.

11. Implant selon l'une des revendications 2 à 10,
**caractérisé en ce que**
l'évidement (82) comporte au moins une saillie (90) et une région (88) qui va en se rétrécissant à l'extrémité de l'évidement, et
dans la situation de prépositionnement, le premier élément (80 ; 210) est maintenu de manière lâche entre la saillie (90) et la région (88) qui va en se rétrécissant.

12. Implant selon l'une des revendications 2 à 11,
**caractérisé en ce que** l'évidement comporte au moins deux saillies (46, 48 ; 114 ; 162, 164 ; 206 ; 208), et
dans la situation de prépositionnement, le premier élément (40 ; 108 ; 154) est maintenu de manière lâche entre au moins deux saillies.

13. Implant selon l'une des revendications 2 à 12,
**caractérisé en ce que**
l'évidement (122) comporte au moins une saillie (134),
le premier élément (128) comporte au moins deux renfoncements (138 ; 140) pour recevoir la saillie (134),
dans la situation de prépositionnement, le premier élément (128) est maintenu de manière lâche au moyen de la saillie (134) qui s'engage dans l'un desdits au moins deux renfoncements (138), et
dans la situation de positionnement final, la saillie (134) est positionnée dans un autre desdits au moins deux renfoncements (140).

14. Implant selon l'une des revendications précédentes,
**caractérisé en ce que**
dans la situation de positionnement final, le premier élément (40 ; 60, 62 ; 80 ; 108 ; 128 ; 154 ; 192 ; 210) est relié au second élément (6 ; 44 ; 63 ; 84 ; 100 ; 120 ; 150 ; 190 ; 214) par coincement et/ou au moyen d'une liaison à encliquetage en coopération de forces et en coopération de formes.

15. Implant selon l'une des revendications précédentes,
**caractérisé en ce que** le premier élément (8 ; 80 ; 210) comprend au moins une saillie qui s'engage dans le second élément (6 ; 84 ; 214) dans la situation de positionnement final.

16. Implant selon l'une des revendications précédentes,
**caractérisé en ce que** le premier élément (40 ; 60 ; 80 ; 108 ; 128 ; 154 ; 210) est une bille (40 ; 60) ou une tige (80 ; 108 ; 128 ; 154 ; 210), en particulier une bille métallique ou une tige métallique.

17. Implant selon l'une des revendications 2 à 16,
**caractérisé en ce que**
le premier élément (60, 62) comprend une bille (60) et un bouchon (62) avec un logement pour la bille,
dans la situation de prépositionnement, la bille (60) est maintenue de manière lâche dans le bouchon (62) ou entre le bouchon (62) et l'extrémité de l'évidement, et
dans la situation de positionnement final, le bouchon (62) est maintenu dans l'évidement et la bille (60) est maintenue dans le bouchon (62) par coopération de forces et par coopération de formes.

18. Implant selon l'une des revendications précédentes,
**caractérisé en ce que** le premier élément est une tige (210) avec un tenon d'ancrage (212).

19. Implant selon l'une des revendications précédentes,
**caractérisé en ce que**
le second élément est une coque intérieure (6) d'une prothèse articulaire et le second élément est une plaque de recouvrement (8) de la coque intérieure,
il est prévu un évidement (12) dans la paroi extérieure de la coque intérieure avec une section transversale de forme circulaire, avec une paroi latérale (22) et avec un fond (20) qui devient de plus en plus profond vers le centre de l'évidement,
dans lequel la paroi latérale (22) comporte au moins une saillie (24) dirigée vers le centre de l'évidement,
dans la situation de prépositionnement, la plaque de recouvrement (8) est maintenue de manière lâche par la paroi latérale et par la saillie,
la plaque de recouvrement (8) comporte au moins une came (28) qui, dans la situation de positionnement final, s'engage dans la paroi latérale (22) de l'évidement, et
la plaque de recouvrement (8) est déplaçable par un mouvement rotatif depuis la situation de prépositionnement jusque dans la situation de positionnement final.

20. Implant selon la revendication 19,
**caractérisé en ce qu'**il est prévu au moins un logement (30) pour la came (28) dans la situation de prépositionnement dans la paroi latérale (22) de l'évidement.

21. Implant selon la revendication 19 20,
**caractérisé en ce que**
à la transition depuis la paroi latérale (22) vers le fond (20), l'évidement (12) comporte une rainure (26) qui s'étend autour de l'évidement,
et/ou **en ce que** la paroi latérale (22) de l'évidement comporte un guidage d'introduction (32) pour amener le premier élément (8) jusque dans la situation de prépositionnement.

22. Agencement constitué d'un implant selon l'une des revendications précédentes et d'un emballage dans lequel l'implant est emballé de manière stérilisée au moyen d'un gaz,
**caractérisé en ce que**
les éléments (6, 8 ; 40, 44 ; 60, 62, 63 ; 80, 84 ; 100, 108 ; 120, 128 ; 150, 154 ; 190, 192 ; 210, 214) occupent la situation de prépositionnement dans l'emballage perméable aux gaz et imperméable aux micro-organismes, situation à partir de laquelle ils peuvent être transférés vers la situation de positionnement final après ouverture de l'emballage par des mouvements de rotation et/ou de translation.

23. Procédé pour la réalisation d'un agencement constitué d'un implant selon l'une des revendications 1 à 21, et d'un emballage dans lequel l'implant a été emballé de manière stérilisée, et qui est transportable et stockable avec l'implant,
dans lequel les éléments (6, 8 ; 40, 44 ; 60, 62, 63 ; 80, 84 ; 100, 108 ; 120, 128 ; 150, 154 ; 190, 192 ; 210, 214) qui constituent l'implant sont tout d'abord amenés dans la situation de prépositionnement, puis ils sont amenés dans l'emballage dans la situation de prépositionnement et sont enfin stérilisés avec un gaz.

24. Procédé selon la revendication 23, **caractérisé en ce que** les éléments (6, 8 ; 40, 44 ; 60, 62, 63 ; 80, 84 ; 100, 108 ; 120, 128 ; 150, 154 ; 190, 192 ; 210, 214) qui constituent l'implant demeurent dans l'emballage dans la situation de prépositionnement lors du transport et du stockage.

25. Procédé selon la revendication 23 24,
**caractérisé en ce que** la stérilisation avec un gaz est exécutée avec de l'oxyde d'éthylène.

26. Procédé selon l'une des revendications 23 à 25,
**caractérisé en ce que** l'emballage est refermé, après introduction des éléments (6, 8 ; 40, 44 ; 60, 62, 63 ; 80, 84 ; 100, 108 ; 120, 128 ; 150, 154 ; 190, 192 ; 210, 214) et avant la stérilisation aux gaz avec une membrane perméable aux gaz et imperméable aux micro-organismes.
